# EUROPEAN PATENT SPECIFICATION

(11) **EP 1 644 563 B1**
(45) Date of publication and mention of the grant of the patent: **04.03.2009**
(21) Application number: 04740553.5
(22) Date of filing: 11.06.2004
(51) Int. Cl.: D04H 1/42

(54) **WETTABLE AND DISINTEGRABLE COSMETIC ARTICLE**
BENETZBARES UND DESINTEGRIERBARES KOSMETISCHES ERZEUGNIS
ARTICLE COSMETIQUE APTE AU MOUILLAGE ET A LA DESINTEGRATION

(30) Priority: 03.07.2003 FR 0308102; 16.07.2003 US 487254 P
(43) Date of publication of application: 12.04.2006
(73) Proprietor: L'ORÉAL, 75008 Paris (FR)
(72) Inventor: MARERI, Pascale, F-75013 Paris (FR); FONTAINE, Michel, F-75008 Paris (FR)
(74) Representative: Julio, Charlotte
(86) International application number: PCT/EP2004/007190
(87) International publication number: WO 2005/003423

(56) References cited:
- EP-A- 1 010 783
- EP-A- 1 166 707
- US-A- 5 208 104
- US-A1- 2003 073 362

## Description

The present invention relates to a cosmetic or care article. On account of their consistency and their ease of use, cosmetic articles of this type are generally known as supports or "wipes". An advantageous use of this type of article is for it to be wetted before use and then to be used as a means of cleansing and/or of applying a care substance to the skin or the hair, although other uses are also possible. Specifically, this article may also be employed for household use in dusting or waxing applications.

Individual cleansing supports have been known for a long time. These supports are generally classified among the following two main types: "wet wipes" or "dry wipes". The invention relates to the field of "dry wipes", preferably for use in wetted form. For example, the teaching of document US 4 303 543 discloses a nonwoven fibrous support impregnated with an aqueous solution comprising a surfactant and a softener. The nonwoven fibrous support is then dried in the form of a dry wipe. Finally, when it is desired to use this dry wipe, the user simply moistens the said dry wipe with water and can then wash his skin or hair and/or disentangle his hair with the wipe.

One subject of the invention is a single-use cosmetic article comprising at least one fibrous support extending substantially in at least one plane, and made of at least one material chosen such that:
i) when the support is placed in contact with water at a first temperature (T0), it at least partially shrinks and becomes elastic in at least one direction of the said plane, and such that
ii) when the support is placed in contact with water at a second temperature (T1), higher than the first temperature (T0), it dissolves so as to become at least partially disintegrated.

To make a nonwoven support according to the invention from water-soluble polyvinyl alcohol fibres, the process is performed in three successive steps: formation of the web, consolidation and then finishing. However, in certain recent technological developments, these steps tend to overlap partially or even completely, with the aim of obtaining new properties for the support.

Firstly, the web of fibres is derived from balls of chopped fibres or from filaments. It may be obtained, for example, via one of the following three methods: via the dry route, via the molten route or via the wet route. In particular, the dry route may be performed as follows. A mechanical process converts the entangled fibres into a "fabric" of parallel fibres. This begins by opening the balls of fibres, which are then blended and then conveyed to a carding machine via a conveyor belt. This machine is formed from one or more rotary drums with thin wires or teeth that comb the fibres.

The weight per unit area envisaged for the nonwovens and the desired orientation of the fibres are important factors. The parameters of the machine derive therefrom. Thus, the fibres may be oriented in the length of the web (i.e. in the machine direction, which also corresponds to a main axis of elongation of the fibres of the support finally obtained). The mechanical properties are generally better in the machine direction than in the transverse direction. However, the machine parameters may be adjusted so as to reduce this anisotropy. Along with the composition of the web, the speed of formation may be modified as a function of the desired properties.

In the present case, to make a nonwoven according to the invention, the process begins with short polyvinylalcohol fibres about 1 cm long. In particular, the fibres used in the invention are water-soluble fibres of the Kuralon II^{®} type from the company Kuraray. In particular, the fibres chosen in this category are those with a dissolution temperature of greater than 20°C, for example about 55°C or 40°C depending on the working temperatures that are then desired. The chosen fibres are, for example, of the type described in document EP-B-0 636 716.

The web obtained is not solid enough to be used in its present form. Consolidation is thus necessary in order to hold the web together. This is a very important step in the production of nonwovens. Specifically, the final properties differ according to the chosen method. For example, the web may be consolidated via chemical bonding, thermal bonding or mechanical bonding. The oldest technique for consolidating nonwovens is mechanical bonding, for example by needle-bonding or entanglement with jets of water. This bonding physically entangles the fibres and the friction of the fibres against each other reinforces the web.

In the case of entanglement with jets of water, the fibres are subjected to the impact of very fine jets of water at high pressure and become entangled. The pressure of the jets determines the strength of the nonwoven that it is desired to obtain. The nonwovens obtained by this method have specific properties; great softness of feel, a very attractive texture and high strength. In general, the jets of water are perpendicular to the nonwoven.

Finally, the finishing of the web, before or after consolidating the web, may be obtained by adding certain products chosen from a large number of chemical substances and may thus give the nonwoven specific properties according to the applications: hydrophobic, porous, antistatic, absorbent, conductive, flame-retardant, air-permeable. Different treatments are applied, for instance coating, printing, flocking, dyeing or combination with other materials to form more complex supports.

Finally, the nonwoven web is completed and rolled up. It may be subjected to new treatments and fashioned in the form of supports. The final form of the supports is obtained after treatment in transformers, which split, cut, fold, sew, impregnate and back the web as desired. For example, for the purpose of a cosmetic use, the support is at least partially impregnated with a cosmetic product, preferably an anhydrous product, especially a makeup-removing product.

In the invention, the nonwoven supports obtained have a weight per unit area of about 50 g/m².

The support formed from such a nonwoven is preferably packaged dry in order subsequently to be able to be used moistened. When the support is wetted, at a first temperature (T0) for its future use, the shrinkage of the support on itself is at least 45% of the lengths and/or widths of the support when it is defined in a plane. Preferably, the shrinkage is at least 60% in one direction of the said plane. In particular, the support is wetted at this stage at a temperature of less than 40°C, which corresponds to a temperature of dissolution of the support.

Due to the shrinkage of the support along its length and/or its width, a reduction in the area formed in the plane by the support, by a factor of at least 4, is observed in parallel.

The thickness of the support may optionally be modified by passing from the first state, which is dry overall, to the second wet, i.e. moistened, state. In the second state, the thickness may be found to have increased. In parallel, the coarseness of the surface of the support is greater in the wet state than in the dry state. This coarseness of the support gives advantageous mechanical properties for cleansing the skin.

The support develops the above physical characteristics when it is wetted using a solution whose temperature is below the dissolution temperature of the fibres constituting it. In the above example, the dissolution temperature of the support is greater than 20°C and is defined at about 40°C.

When the support is wetted with a solution at a temperature above its dissolution temperature, in this instance about 40°C for the above example, then the support rapidly dissolves. It may thus be readily disposed of without, however, creating additional waste.

In parallel with its shrinkage, the support also becomes more elastic when it is in a wetted state than when it is in its initial dry state. This elasticity is reflected by an elongation at break for the nonwovens according to the invention comprising a main axis of elongation of the fibres. This elongation at break is at least fivefold or even tenfold in the direction of the main axis of elongation of the fibres in the wet state relative to the elongation at break obtained when the article is dry.

The support is anisotropic. Specifically, the elongation at break in a direction orthogonal to the main axis of elongation of the fibres decreases on passing from the first dry state to the second wet state, in contrast with the increasing elongation observed in the direction parallel to the main axis of elongation of the fibres.

The supports made according to the invention, once wetted a first time, so as to change from the first state to the second state, can no longer regain their size characteristics or their mechanical properties associated with their first state. The change from the first state to the second state is irreversible. However, the support that has been wetted to the point of reaching its second state becomes stiff if it is not dissolved and is left in the open air. This stiffened state is reversible, and simple rewetting of the support allows it to regain the characteristics observed in the second state, especially its elasticity. The support can thus, nevertheless, be reusable for application to the skin or the hair, even after the first wetting, on condition that the wetting temperature is still less than the dissolution temperature of the support.

The support according to the invention may be cut to the shape of a flat support, of rectangular, oval or circular shape, but it may also be in different forms and cutouts, for example in the form of a wipe, a glove, a pad or an applicator tip. The support may be one-ply or multi-ply and may also be combined with other types of support, for example in combination with a sponge, a fabric or permeable or impermeable supports.

The support according to the present invention may have numerous uses as a function of the ingredients incorporated into the support. More particularly, the invention relates to cosmetic articles in which a surfactant or any other cosmetic component and/or care component is present in solid form, for example in the form of a powder of a foaming or makeup composition. These articles may also comprise liquid, for example encapsulated, such that this liquid can wet the cosmetic article, and thus the surfactant and/or the other cosmetic components, when the capsules are broken.

For example, this type of support may be used to apply a foundation, a skincare product, a haircare product (for example a shampoo, a conditioning balm, a disentangling bath or a dye), a self-tanning composition, an antisun composition, a makeup-removing product, fragrance, an anti-mosquito product, an anti-wrinkle or anti-acne treatment, or any other cosmetic and/or care product.

The support may also comprise fatty substances, for example products that are soluble only in oils, such as makeup compositions, or softeners, or alternatively compositions comprising sunscreens that are only soluble in oil; in this case, the components are encapsulated.

These supports may be presented in various types of packaging. For example, they may be compressed, optionally folded up and stacked on top of each other in a flexible plastic sachet so as to be easily transportable. This type of flexible plastic sachet comprises an opening via which the supports may be removed one after the other. Before the very first use, this opening is sealed and hermetically closed with a film covering this opening and comprising at least one adhesive periphery to ensure leaktightness around this opening. After the first use, the film may optionally be replaced on the opening or may be permanently discarded.

If necessary, the opening may be surrounded by additional flexible tabs extending along the adjacent edges of the opening. This thus gives an opening with flexible edges that can be separated during the removal of the supports contained in the sachet.

The supports may be folded in different ways. For example, the supports may be intercalated with each other such that the extraction of the top support through the opening entrains at least part of the support lying just underneath to rest outside the opening, and thus facilitate its subsequent extraction from the sachet.

As a variant, each support may be packaged in an individual sachet, such that the opening of the sachet may be obtained by tearing the sachet.

Alternatively, the supports may be conditioned in rigid plastic boxes and other types of boxes that are compatible with the structure of the supports and the chemical compositions they contain.

When it is desired to use the support as a wipe for cleansing the face or removing makeup therefrom, the wipe is then impregnated with a liquid or solid composition, for example in the form of a powder, comprising a foaming surfactant. Various types of surfactant may be incorporated, such as anionic, cationic or amphoteric surfactants. The impregnated liquid or solid composition may also comprise one or more softeners, vitamins, a fragrance, neutralizers and other types of active agent, preferably hydrophilic active agents and preserving agents. To obtain the foaming effect, water may then be supplied in a large amount when the wipe is used, and finally for rinsing after use.

In general terms, a subject of the invention is also a process of cosmetic treatment using an article as made above, and consisting in:
i) impregnating the said article with water at a first temperature (T0);
ii) placing at least one face of the article impregnated with the said cosmetic composition in contact with a surface to be treated, especially the skin; and
iii) placing the said article in contact with water at a second temperature (T1) so as to dissolve the support and at least partially disintegrate the said article.

The nonwoven support made with polyvinyl alcohol (PVA) fibres was tested in "dry" form and in "wet" form, i.e. after a residence time of one hour in water. The mechanical properties were tested in the direction parallel and transverse to the main axis of elongation of the fibres of the support. The results presented are means performed on groups of five identically tested supports. The supports tested are rectangles.

These supports are defined according to two perpendicular directions of a plane, for example a length and a width. They moreover have a defined thickness orthogonal to this plane. A thickness is, for example, between 0.1 and 10 mm. The "dry" thickness of these supports is preferably less than 1 mm. As a result, the supports have length and width dimensions that are markedly greater than this thickness. Even in "wet" form, in which the supports gain slightly in thickness, they still have a thickness that is markedly less than their length and width dimensions.

Two types of support were considered. The first supports have a defined length parallel to the main axis of elongation of the fibres; in this case, they have an initial length of 250 mm and an initial width of 50 mm. The second supports have a defined length orthogonal to the main axis of elongation of the fibres; in this case, they have an initial length of 190 mm and an initial width of 50 mm. The results obtained are collated in the table below.

| | First supports | | Second supports | |
|---|---|---|---|---|
| | "Parallel direction (//)" | | "Transverse direction (⊥)" | |
| | Length | Width | Length | Width |
| "Dry" nonwoven | 250 mm | 50 mm | 190 mm | 50 mm |
| "Wet" nonwoven | 110 mm | 20 mm | 100 mm | 27 mm |
| **Shrinkage** | **56%** | **60%** | **47%** | **46%** |

The maximum force and the elongation at break of these various types of nonwoven support were tested, in the dry and then wet states, respectively, by exerting tensile forces at the ends of the supports, such that the tensile forces exerted were parallel to the length of these supports. The results are presented in the table below.

| | First supports | | Second supports | |
|---|---|---|---|---|
| | "Parallel direction (//)" | | "Transverse direction (⊥)" | |
| | Maximum force | Elongation at break | Maximum force | Elongation at break |
| "Dry" nonwoven | 39 N | 13% | 10.1 N | 120% |
| "Wet" nonwoven | 42 N | 161% | 5.5 N | 79.7% |
| **Change** | **+7%** | **×12** | **-45%** | **-33%** |

## Claims

1. Single-use cosmetic article comprising at least one fibrous support, the support being at least partially impregnated with an anhydrous or encapsulated cosmetic product, the support extending substantially in at least one plane, and made of at least one material chosen such that:
i) when the support is placed in contact with water at a first temperature (T0), it at least partially shrinks and becomes elastic in at least one direction of the said plane, and such that
ii) when the support is placed in contact with water at a second temperature (T1), higher than the first temperature (T0), it dissolves so as to become at least partially disintegrated, **characterized in that** the support comprises water-soluble polyvinyl alcohol (PVA) fibres.

2. Article according to Claim 1, **characterized in that** the support is at least partially impregnated with a cosmetic product, which is preferably anhydrous, especially a makeup-removing product.

3. Article according to Claim 1 or 2, **characterized in that,** when it is in contact with water at the first temperature (T0), the support shrinks by at least 45% and preferably by at least 60% in one direction of the said plane.

4. Article according to one of Claims 1 to 3, **characterized in that** the first temperature (T0) is less than or equal to 40°C.

5. Article according to one of Claims 1 to 4, **characterized in that** the second temperature (T1) is greater than or equal to 20°C.

6. Article according to one of Claims 1 to 5, **characterized in that** the fibrous support is a nonwoven and comprises a main axis of elongation of the fibres.

7. Article according to the preceding claim, **characterized in that,** when it is placed in contact with water at a first temperature (T0), its elongation at break in the direction of the main axis of elongation of the fibres is at least five and preferably at least ten times its elongation at break when the article is dry.

8. Article according to either of Claims 6 and 7, **characterized in that,** when it is placed in contact with water at a first temperature (T0), its elongation at break perpendicular to the main axis of elongation of the fibres is smaller than its elongation at break when the article is dry.

9. Article according to one of Claims 1 to 8, **characterized in that** the wetting of the article results in irreversible changes.

10. Article according to one of Claims 1 to 9, **characterized in that,** in the wetted state at the first temperature (T0), the surface state of the article is coarser than in the dry state.

11. Article according to any one of the preceding claims, **characterized in that** it is configured in the form of a wipe, a pad, an applicator tip or a glove.

12. Process of cosmetic treatment using an article according to any one of Claims 1 to 11, the said article being preimpregnated with an essentially anhydrous cosmetic composition, the said process consisting in:
i) impregnating the said article with water at a first temperature (T0);
ii) placing at least one face of the article impregnated with the said cosmetic composition in contact with a surface to be treated, especially the skin; and
iii) placing the said article in contact with water at a second temperature (T1) so as to dissolve the support and at least partially disintegrate the said article.

## Patentansprüche

1. Kosmetischer Einwegartikel, der mindestens einen faserigen Träger umfasst, wobei der Träger mindestens teilweise mit einem wasserfreien oder verkapselten kosmetischen Produkt imprägniert ist, wobei sich der Träger im Wesentlichen in mindestens einer Ebene erstreckt und aus mindestens einem Material hergestellt ist, das so ausgewählt ist, dass:
i) der Träger, wenn er bei einer ersten Temperatur (T0) mit Wasser in Kontakt gebracht wird, mindestens teilweise schrumpft und in mindestens einer Richtung dieser Ebene elastisch wird, und dass
ii) der Träger, wenn er bei einer zweiten Temperatur (T1), die oberhalb der ersten Temperatur (T0) liegt, mit Wasser in Kontakt gebracht wird, sich so auflöst, dass er mindestens teilweise zerfällt, **dadurch gekennzeichnet, dass** der Träger wasserlösliche Polyvinylalkohol-Fasern (PVA-Fasern) umfasst.

2. Artikel nach Anspruch 1, **dadurch gekennzeichnet, dass** der Träger mindestens teilweise mit einem kosmetischen Produkt, das vorzugsweise wasserfrei ist, insbesondere mit einem Make-upentfernenden Produkt, imprägniert ist.

3. Artikel nach Anspruch 1 oder 2, **dadurch gekennzeichnet, dass** der Träger um mindestens 45 % und vorzugsweise mindestens 60 % in einer Richtung dieser Ebene schrumpft, wenn er bei der ersten Temperatur (T0) mit Wasser in Kontakt ist.

4. Artikel nach einem der Ansprüche 1 bis 3, **dadurch gekennzeichnet, dass** die erste Temperatur (T0) kleiner als oder gleich 40 °C ist.

5. Artikeln nach einem der Ansprüche 1 bis 4, **dadurch gekennzeichnet, dass** die zweite Temperatur (T1) größer als oder gleich 20 °C ist.

6. Artikeln nach einem der Ansprüche 1 bis 5, **dadurch gekennzeichnet, dass** der faserige Träger ein Vliesstoff ist und eine Hauptachse der Dehnung der Fasern aufweist.

7. Artikel nach dem vorhergehenden Anspruch, **dadurch gekennzeichnet, dass** seine Bruchdehnung in Richtung der Hauptachse der Dehnung der Fasern, wenn er bei einer ersten Temperatur (T0) mit Wasser in Kontakt gebracht wird, mindestens fünfmal und vorzugsweise mindestens zehnmal so groß ist wie seine Bruchdehnung, wenn der Artikel trocken ist.

8. Artikeln nach einem der Ansprüche 6 und 7, **dadurch gekennzeichnet, dass** seine Bruchdehnung senkrecht zur Hauptachse der Dehnung der Fasern, wenn er bei einer ersten Temperatur (T0) mit Wasser in Kontakt gebracht wird, kleiner als seine Bruchdehnung ist, wenn der Artikel trocken ist.

9. Artikel nach einem der Ansprüche 1 bis 8, **dadurch gekennzeichnet, dass** die Befeuchtung des Artikels zu irreversiblen Änderungen führt.

10. Artikel nach einem der Ansprüche 1 bis 9, **dadurch gekennzeichnet, dass** der Zustand der Oberfläche des Artikels im befeuchteten Zustand bei der ersten Temperatur (T0) rauer ist als im trockenen Zustand.

11. Artikel nach einem der vorhergehenden Ansprüche, **dadurch gekennzeichnet, dass** er in der Form eines Wischtuchs, eines Kissens, einer Applikatorspitze oder eines Handschuhs konfiguriert ist.

12. Verfahren zur kosmetischen Behandlung unter Verwendung eines Artikels nach einem der Ansprüche 1 bis 11, wobei der Artikel mit einer im Wesentlichen wasserfreien Zusammensetzung vorimprägniert ist, wobei das Verfahren besteht aus
i) Imprägnieren dieses Artikels mit Wasser bei einer ersten Temperatur (T0);
ii) Inkontaktbringen mindestens einer Fläche des Artikels, der mit der kosmetischen Zusammensetzung imprägniert ist, mit einer zu behandelnden Oberfläche, insbesondere der Haut, und
iii) Inkontaktbringen des Artikels mit Wasser bei einer zweiten Temperatur (T1), um den Träger aufzulösen und den Artikel mindestens teilweise zu zersetzen.

## Revendications

1. Article cosmétique à usage unique comprenant au moins un support fibreux, le support étant au moins partiellement imprégné d'un produit cosmétique anhydre ou encaspulé, le support s'étendant sensiblement dans au moins un plan et étant réalisé dans au moins un matériau choisi de telle sorte que :
i) lorsque le support est placé au contact de l'eau à une première température (T0), il se rétracte au moins partiellement et devient élastique dans au moins une direction dudit plan, et de telle sorte que
ii) lorsque le support est placé au contact de l'eau à une deuxième température (T1), supérieure à la première température (T0), il se dissout de manière à se désintégrer au moins partiellement, **caractérisé en ce que** le support comprend des fibres d'alcool polyvinylique (PVA) hydrosolubles.

2. Article selon la revendication 1, **caractérisé en ce que** le support est au moins partiellement imprégné d'un produit cosmétique, qui est de préférence anhydre, en particulier d'un produit démaquillant.

3. Article selon la revendication 1 ou 2, **caractérisé en ce que,** lorsqu'il est au contact de l'eau à la première température (T0), le support se rétracte d'au moins 45 % et de préférence d'au moins 60 % dans une direction dudit plan.

4. Article selon l'une des revendications 1 à 3, **caractérisé en ce que** la première température (T0) est inférieure ou égale à 40 °C.

5. Article selon l'une des revendications 1 à 4, **caractérisé en ce que** la deuxième température (T1) est supérieure ou égale à 20 °C.

6. Article selon l'une des revendications 1 à 5, **caractérisé en ce que** le support fibreux est un non-tissé et comprend un axe d'allongement principal des fibres.

7. Article selon la revendication précédente, **caractérisé en ce que,** lorsqu'il est placé au contact de l'eau à une première température (T0), son allongement à la rupture dans la direction de l'axe d'allongement principal des fibres est égal à au moins cinq fois et de préférence à au moins dix fois son allongement à la rupture lorsque l'article est sec.

8. Article selon l'une ou l'autre des revendications 6 et 7, **caractérisé en ce que,** lorsqu'il est placé au contact de l'eau à une première température (T0), son allongement à la rupture perpendiculairement à l'axe d'allongement principal des fibres est inférieur à son allongement à la rupture lorsque l'article est sec.

9. Article selon l'une des revendications 1 à 8, **caractérisé en ce que** l'humidification de l'article conduit à des changements irréversibles.

10. Article selon l'une des revendications 1 à 9, **caractérisé en ce que,** à l'état humidifié à la première température (T0), l'état de surface de l'article est plus rugueux qu'à l'état sec.

11. Article selon l'une quelconque des revendications précédentes, **caractérisé en ce qu'**il est configuré sous forme d'une lingette, d'un tampon, d'un embout d'applicateur ou d'un gant.

12. Procédé de traitement cosmétique au moyen d'un article selon l'une quelconque des revendications 1 à 11, ledit article étant pré-imprégné d'une composition cosmétique essentiellement anhydre, ledit procédé consistant à :
i) imprégner ledit article d'eau à une première température (T0) ;
ii) placer au moins une face de l'article imprégnée de ladite composition cosmétique au contact d'une surface à traiter, en particulier la peau ; et
iii) placer ledit article au contact de l'eau à une deuxième température (T1) de manière à dissoudre le support et à désintégrer au moins partiellement ledit article.
